(19) Europäisches Patentamt
European Patent Office
Office européen des brevets

(11) Publication number: **0 347 852 B1**

(12) # EUROPEAN PATENT SPECIFICATION

(45) Date of publication of patent specification: **23.08.95**

(51) Int. Cl.[6]: **C07D 473/00**, C07D 471/04, A61K 31/52

(21) Application number: **89111230.2**

(22) Date of filing: **20.06.89**

(54) **Novel neplanocin derivatives.**

(30) Priority: **20.06.88 US 209245**

(43) Date of publication of application:
**27.12.89 Bulletin 89/52**

(45) Publication of the grant of the patent:
**23.08.95 Bulletin 95/34**

(84) Designated Contracting States:
**AT BE CH DE ES FR GB GR IT LI LU NL SE**

(56) References cited:
**EP-A- 0 262 876**
**FR-A- 2 500 838**
**FR-A- 2 580 283**
**GB-A- 2 021 582**

(73) Proprietor: **MERRELL DOW PHARMACEUTICALS INC.**
**2110 East Galbraith Road,**
**P.O. Box 156300**
**Cincinnati**
**Ohio 45215-6300 (US)**

(72) Inventor: **Jarvi, Esa T.**
**3953 St. Johns Terrace**
**Cincinnati, Ohio 45236 (US)**
Inventor: **McCarthy, James R.**
**6448 Foxview Place**
**West Chester, Ohio 45069 (US)**
Inventor: **Prakash, Nellikunja Jaya**
**10871 Wengate Lane**
**Cincinnati, Ohio 45241 (US)**

(74) Representative: **VOSSIUS & PARTNER**
**Postfach 86 07 67**
**D-81634 München (DE)**

EP 0 347 852 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

S-Adenosyl-L-methionine (AdoMet) dependent transmethylation reactions have been implicated in a variety of biological processes related to viral growth and replication, viral transformation of cells, growth of malignant cells, and processes such as chemotaxis and secretion [See P. M. Ueland, Pharm. Reviews, 34, 223 (1982)]. In general, these transmethylation reactions are catalyzed by various transmethylases which utilize AdoMet as a methyl-donor substrate in the methylation of a number of methyl-acceptor substrates such as catechols; norepinephrine; histamine; serotonin; tryptamine; membrane phospholipids; lysyl, arginyl, histidyl, aspartyl, glutamyl, and carboxyl groups of certain proteins; tRNA and mRNA; and DNA. These various transmethylases produce S-Adenosyl-L-Homocysteine (AdoHcy) as a byproduct upon transfer of a methyl group from AdoMet to the appropriate methyl-acceptor substrate.

AdoHcy has been shown to be a potent feed-back inhibitor of the AdoMet-dependent transmethylation reactions. This feedback inhibition of the transmethylases is controlled by the biodegradation of AdoHcy by S-Adenosyl-L-Homocysteine Hydrolase which provides a homeostatic control on the tissue levels of AdoHcy. The activity of S-Adenosyl-L-Homocysteine Hydrolase is generally considered by those skilled in the art to play an important role in regulating the tissue levels of AdoHcy and thereby controlling the activity of the AdoMet dependent transmethylation reactions.

In GB-A 2 021 582 neplanocins, including neplanocin A of the formula

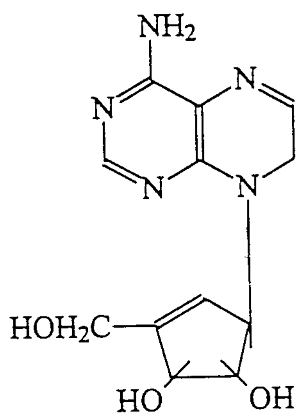

are described as having anti-tumor activity.

The compounds of the present invention are inhibitors of S-Adenosyl-L-Homocysteine Hydrolase. These compounds therefore inhibit the naturally-occurring biodegradation of AdoHcy and result in elevated tissue levels of AdoHcy. Elevated levels of AdoHcy in turn provide an endogenous feed-back inhibition of various AdoMet dependent transmethylation reactions which are associated with biological processes related to viral growth and replication, viral transformation of cells, growth of malignant cells, and processes such as chemotaxis and secretion. The compounds of the present invention are therefore useful as inhibitors of these biological processes and useful in an end use application as therapeutic agents in the treatment of patients afflicted with various pathological conditions in which these processes are implicated, such as, viral infections and neoplastic disease states.

The present invention relates to novel neplanocin derivatives which are useful as inhibitors of S-Adenosyl-L-Homocysteine Hydrolase and are useful as anti-viral and anti-neoplastic agents.

The present invention provides novel compounds of the formula (1)

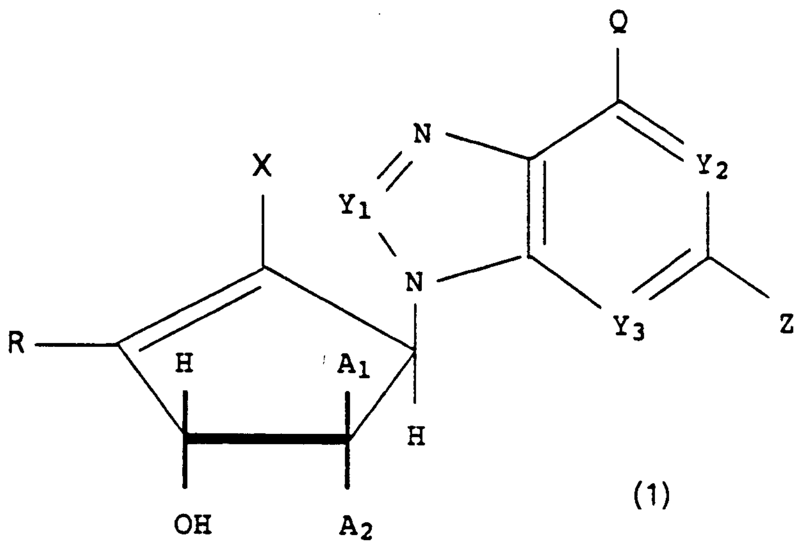

(1)

wherein

| | |
|---|---|
| R | is hydrogen or a $C_1$-$C_4$ alkyl optionally substituted with a hydroxy group, |
| X | is halogen, |
| $A_1$ and $A_2$ | are each independently hydrogen, halogen, or hydroxy with the provisos that where $A_1$ is hydroxy, $A_2$ is hydrogen, and that where $A_2$ is hydroxy, $A_1$ is hydrogen, |
| $Y_1$ | is nitrogen, a CH group, a CF group, a CCl group, a CBr group or a $CNH_2$ group, |
| $Y_2$ and $Y_3$ | are each independently nitrogen or a CH group, |
| Q | is $NH_2$, NHOH, $NHCH_3$, or hydrogen, and |
| Z | is hydrogen, halogen, or $NH_2$; |

and pharmaceutically-acceptable salts thereof.

The present invention also provides a method of inhibiting AdoMet-dependent transmethylation activity in a patient in need thereof comprising administration of an effective inhibitory amount of a compound of formula (1).

Another embodiment of the present invention is the use of a compound of formula (1) for the preparation of a pharmaceutical composition for treating a patient afflicted with a neoplastic disease state or for controlling the growth of a neoplasm in a patient afflicted with a neoplastic disease state comprising administration of an effective antineoplastic amount of a compound of formula (1).

A further embodiment of the present invention is the use of a compound of formula (1) for the preparation of a pharmaceutical composition for treating a patient afflicted with a viral infection or for controlling a viral infection in a patient afflicted therewith comprising administration of an effective antiviral amount of a compound of formula (1).

As used herein, the term "halogen" refers to a fluorine, chlorine, bromine, or iodine atom and the term "nitrogen" refers to a trivalent nitrogen atom attached to two radicals. The term "$C_1$-$C_4$ alkyl" refers to a saturated straight or branched chain hydrocarbyl radical of one to four carbon atoms.

The neplanocin derivatives of formula (1) can be prepared by utilizing procedures and techniques well known and appreciated by one skilled in the art.

A general synthetic procedure for the preparation of compounds of formula (1) is set forth in Scheme A. In the following schemes all substituents, unless otherwise indicated, are as previously defined. In addition, the term "L" refers to a leaving group such as an o-tosyl group.

SCHEME A

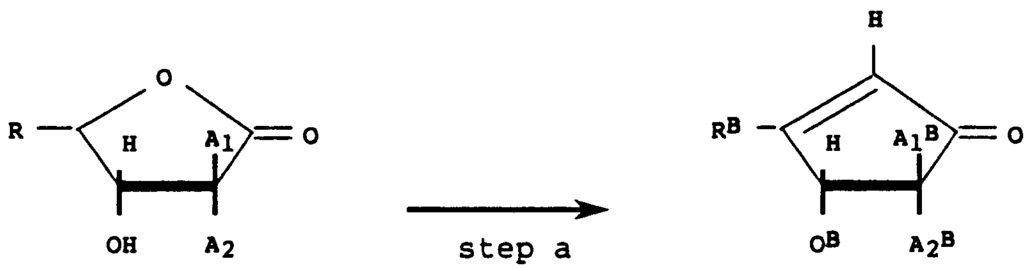


(2) (3)

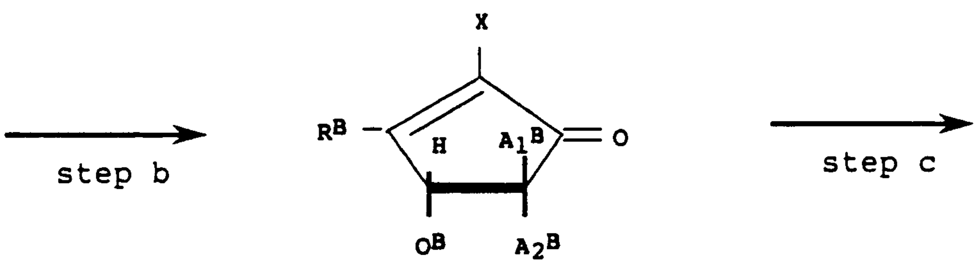


(4)

Scheme A (cont'd)

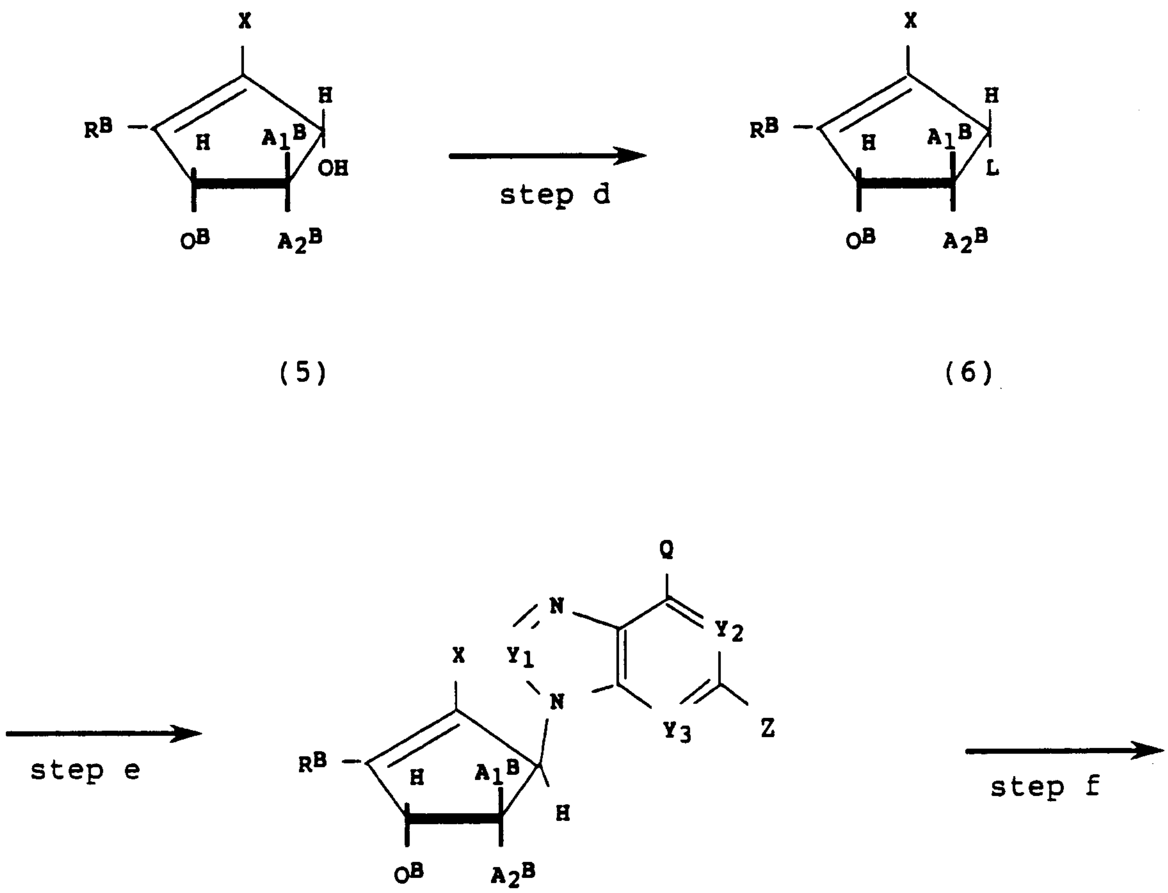


(5)

(6)

(7)

Scheme A (cont'd)

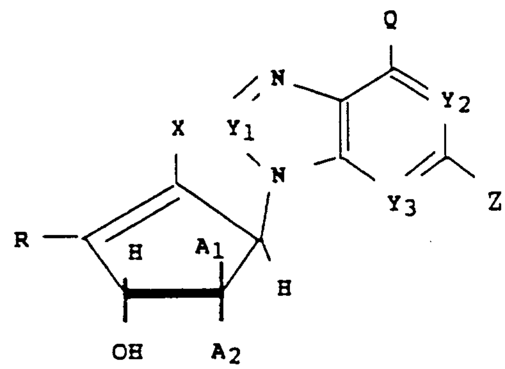

(8)

In step a, ribonolactone derivatives (2) are converted to the corresponding appropriately blocked cyclopentenone derivatives (3) according to procedures which are well known and appreciated by those skilled in the art. For example, where compounds of the present invention are desired wherein R is hydrogen, the appropriately blocked cyclopentenone derivatives (3) can be prepared according to methods analogous to that described by Borcherding et al., J. Org. Chem. 1987, 52, 5457. Where compounds of the present invention are desired wherein R is hydroxymethyl, the appropriately blocked cyclopentenone derivative (3) can be prepared according to methods analogous to that described by Lim and Marquez, Tetrahedron Lett., **1983**, 24, 5559. Other compounds of the present invention can be prepared by methods analogous to the above-identified methods or by other methods well known and appreciated by those skilled in the art.

The reactive hydroxy groups of the appropriate cyclopentenone derivatives (3), including the 3-hydroxy, any 2-hydroxy, and any hydroxy substituents of the R group, are blocked with standard blocking agents well known in the art. These blocking groups can be conventional hydroxy protecting groups as are well known and appreciated by those skilled in the art. $O^B$, $A_1{}^B$, $A_2{}^B$ and $R^B$ in Scheme A represent the 3'-hydroxy, $A_1$, $A_2$, and R groups as herein defined blocked with a blocking group where appropriate.

The selection and utilization of particular blocking groups are well known to those of ordinary skill in the art. In general, blocking groups should be selected which adequately protect the hydroxy groups in question during subsequent synthetic steps and which are readily removable under conditions which will not cause degradation of the desired product.

Examples of suitable hydroxy protecting groups are $C_1$-$C_6$ alkyl, tetrahydropyranyl, methoxymethyl, methoxyethoxy-methyl, t-butyl, benzyl, and triphenylmethyl. The term $C_1$-$C_6$ alkyl refers to a saturated hydrocarbyl radical of one to six carbon atoms of straight, branched, or cyclic configuration. The preferred blocking groups for the 3-hydroxy and for $A_2$ (wherein $A_2$ is hydroxy) include 2,3-0-isopropylidene (formed by reacting the appropriate compound with acetone), 2,3-O-cyclohexylidene (formed by reacting the appropriate compound with cyclohexanone and $H_2SO_4$) and alkoxymethylidene (formed by reacting the appropriate compound with trialkylorthoformate). The preferred blocking group for any hydroxy substituent of R is benzyl (formed by reacting the appropriate compound with benzyl bromide and sodium hydride in dimethylformamide).

In step b, the appropriately blocked cyclopentenone derivative (3) is halogenated and then dehydrohalogenated to yield the corresponding 5-halo-cyclopentenone derivative (4) by the use of standard procedures such as, for example, the procedures described by Grenier-Loustalot and co-workers in Synthesis, **1976**, p. 33, and by Merritt and Stevens in J. Am. Chem. Soc., **1966**, 88, 1822. the preferred halogenating reagent to effect a fluorination is fluorine gas. The preferred halogenating reagent to effect a chlorination is sulfuryl chloride. After halogenation is complete, the reaction mixture is dehydrohalogenated by treatment with base. The preferred bases for dehydrohalogenation are sodium bicarbonate, sodium

methoxide, triethylamine and 1,8-diazabicyclo[5.4.0]undec-7-ene.

In step c, the 5-halo-cyclopentenone derivative (4) is reduced to the corresponding 5-halo-cyclopenten-1-ol derivative (5). The preferred means of this reduction is by treating (4) with cesium chloride and sodium borohydride.

In step d, the 5-halo-cyclopenten-1-ol derivative (5) is derivatized to provide a leaving group in the 1-position as indicated in (6). Leaving groups are substituents which can be displaced by a nucleophilic moiety with relative ease. Often the nucleophilic substitution occurs through an $S_N2$ type displacement.

The selection and utilization of particular leaving groups are well known to one of ordinary skill in the art. In general, leaving groups should be selected which provide a stable derivative and which can easily be displaced by a nucleophile such as sodium adenine. Examples of suitable leaving groups are p-toluenesulfonyl (tosyl), methanesulfonyl, trifluoromethanesulfonyl, p-nitrobenzenesulfonyl and benzenesulfonyl. The preferred leaving group for the 1-position of 5-halo-cyclopenten-1-ol (5) is the tosyl group [formed by reacting (5) with p-toluenesulfonyl chloride].

In step e, the 5-halo-cyclopentene derivative bearing a leaving group in the 1-position (6) is reacted with the appropriate adenine derivative/analog base in a nucleophilic displacement wherein the base is substituted for the leaving group. For example, (6) can be reacted with sodium adenine to yield the 5′-halo-cyclopenten-1′-adenine derivative (7).

In step f, the hydroxy protecting groups are removed according to conventional procedures and techniques well known and appreciated in the art to yield the desired 5′-halo-cyclopenten-1′-adenine derivative (8). For example, the 2′,3′-0-cyclohexylidene blocking group can be removed by reacting (7) with aqueous hydrochloric acid and a benzyl blocking group can be removed by reacting (7) with $BBr_3$.

Starting materials for use in the general synthetic procedure outlined in Scheme A are readily available to one of ordinary skill in the art. For example, certain starting materials for various compounds of formula (1) are listed in Table 1.

## TABLE 1

### Examples of Starting Materials for Scheme A

Compound of formula (1) wherein

| R | $A_1$ | $A_2$ | $Y_1$ | $Y_2$ | $Y_3$ | Z | Q | Source of Starting Material |
|---|---|---|---|---|---|---|---|---|
| - | H | OH | CH | N | N | Cl | $NH_2$ | 2-Chloroadenine and Tet. Lett. 1977, 3433 |
| - | H | OH | CH | N | N | H | $NH_2$ | Adenine |
| - | H | OH | CH | N | CH | H | $NH_2$ | 3-deazaadenine |
| H | H | OH | - | - | - | - | - | ribonolactone |
| $CH_2OH$ | H | OH | - | - | - | - | - | ribonolactone |

Additional starting materials can be prepared by the use of methods analogous to those described in Table 1 as well as other conventional methods as are well known and appreciated in the art.

The following examples present a typical synthesis as described by Scheme A.

EXAMPLE 1

(-)-9-(5'-CHLORO-TRANS-2',TRANS-3'-DIHYDROXY-CYCLOPENT-4'-ENYL)ADENINE

Step a: (-)-2,3-(Cyclohexylidenedioxy)-4-cyclopentenone

Prepare the title compound according to the procedure of Borcherding et al., J. Org. Chem. 1987, 52, 5457.

Step b: (-)-5-Chloro-2,3-(cyclohexylidenedioxy)-4-cyclopentenone

Prepare the title compound according to the procedure of Grenier-Loustalot, Synthesis **1976**, p.33. Cool a solution of (-)-2,3-(cyclohexylidenedioxy)-4-cyclopentenone [139 milligrams (mg), 0.72 millimoles (mmol)] in dichloromethane [7 milliliters (ml)] in an ice bath under nitrogen. To the cooled solution, add sulfuryl chloride (0.14 ml, 1.44 mmol) over about 10 to 20 seconds. Remove the ice bath and monitor the progress of the reaction by thin-layer chromatography (TLC) using dichloromethane as the developing solvent. After about 2 hours, dilute the reaction mixture with chloroform (5ml) and pour the mixture into a solution of saturated/aqueous sodium bicarbonate (15ml) and water (10ml). Extract the title compound into chloroform ( 50ml; 2 times) and dry the organic layer over anhydrous $MgSO_4$. Evaporate the solvent to dryness *in vacuo* and purify the title compound by flash chromatography using a silica gel column and eluting sequentially with dichloromethane:cyclohexane (1:1) followed by dichloromethane. Evaporate the fractions to dryness to yield a solid (133 mg).
Melting point (mp) 66-68 degrees centigrade (°C); $^1$HNMR ($CDCl_3$, 90MHz) $\delta$ 1.3-1.7 (m, 10H), 4.54 (d, 1H, J = 2.4Hz), 5.18 (d of d, 1H, J = 5.5 and 2.4 Hz), 7.45 (d, 1H, J = 2.4 Hz).

Step c: (-)-5-Chloro-2,3-(cyclohexylidenedioxy)-4-cyclopenten-1-ol

Cool a solution of (-)-5-chloro-2,3-(cyclohexylidenedioxy)-4-cyclopentenone [2.4 grams (g), 10.5 mmol] in methanol (50ml) to 0°C and treat the solution with $CeCl_3 \cdot 7H_2O$ (3.91 g, 10.5 mmol) and $NaBH_4$ (0.48 g, 12.6 mmol). (Note that this procedure requires caution since it results in foaming.) Stir the mixture for 20 minutes (min) and adjust the pH to 7.0 with 1N HCl. Extract the title compound into diethyl ether (200ml) and wash the ether layer with brine. Dry the organic layer over anhydrous $MgSO_4$ and evaporate the solvent to dryness *in vacuo*. Purify the title compound by flash chromatography using a silica gel column and eluting with dichloromethane.

Step d: (-)-5-Chloro-2,3-(cyclohexylidenedioxy)-1-[p-tolylsulfonyl)oxy]cyclopent-4-ene

Treat a solution of (-)-5-chloro-2,3-(cyclohexylidenedioxy)-4-cyclopenten-1-ol (2.3 g, 10 mmol) in dichloromethane (100ml) with p-toluenesulfonyl chloride (3.8 g, 20 mmol) followed by triethylamine (4.0 g, 40 mmol). Stir the mixture at room temperature for about 24 hours. Extract the mixture with $H_2O$ and brine. Dry the organic layer over anhydrous $Na_2SO_4$ and evaporate the solvent to dryness *in vacuo*. Purify the title compound by flash chromatography using a silica gel column and eluting with dichloromethane:hexane (1:1).

Step e: (-)-9-[5'-Chloro-2',3'-(cyclohexylidenedioxy) cyclopent-4'-enyl]adenine

To a solution of (-)-5-Chloro-2,3-(cyclohexylidenedioxy)-1-[p-tolylsulfonyl)oxy]cyclopent-4-ene (1.58 g, 4.1 mmol) in dimethylformamide (DMF; 3 ml) add a solution of sodium adenine in 10 ml of DMF. [Prepare the sodium adenine solution by adding NaH (80%; 0.35 g, 12.3 mmol) to a slurry of adenine (1.66 g, 12.3 mmol) in 10 ml of DMF.] Stir the mixture for about 2 days at 50°C. Evaporate the solvent to dryness *in vacuo* and redissolve the residue in dichloromethane (50ml) and filter. Purify the title compound by flash chromatography using a silica gel column and eluting with dichloromethane:ethanol (9:1).

Step f: (-)-9-[5'-Chloro-trans-2',trans-3'-dihydroxy-cyclopent-4'-enyl]adenine

Mix (-)-9-[5'-chloro-2',3'-(cyclohexylidenedioxy) cyclopent-4'-enyl]adenine (348 mg, 1 mmol) with $H_2O$ (20ml) and add 6N HCl (1ml). Stir the solution at room temperature and monitor the progress of the reaction by TLC [ silica gel, eluting with dichloromethane:ethanol (9:1)]. After removal of the protecting group is

complete (about 6 hours) concentrate the solution and azeotrope with ethanol. Dissolve the resulting solid in $H_2O$ (2ml) and purify by column chromatography by applying the solution to a column of Dowex 1x8-50 ($H^+$-form) resin packed with $H_2O$ and elute with dilute ammonium hydroxide. Evaporate the column fractions containing the title compound to dryness and azeotrope with ethanol to yield the title compound as a white crystalline solid.

In like manner, (-)-9-[5'-chloro-trans-2',trans-3'-dihydroxy-4'-hydroxymethyl-cyclopent-4'-enyl]adenine can be prepared from (-)-2,3-O-(isopropylidenedioxy)-4-benzyloxymethyl-4-cyclopentenone, which in turn is prepared from ribonolactone as described by Lim and Marquez in Tetrahedron Lett. **1983**, 24, 5559. In step f, de-blocking can be accomplished by treatment of the (-)-9-[5'-chloro-2',3'-O-(isopropylidenedioxy)-4'-benzyloxymethyl-cyclopent-4'-enyl]adenine with $BBr_3$ according to standard procedures.

The following specific compounds can be made by procedures analogous to those described above in Example 1:

(-)-9-[5'-chloro-trans-2',trans-3'-dihydroxy-4'-(α-hydroxyethyl)-cyclopent-4'-enyl]adenine
(-)-9-[5'-chloro-cis-2',trans-3'-dihydroxy-4'-hydroxymethyl-cyclopent-4'-enyl]adenine
(-)-9-[5'-chloro-trans-2',trans-3'-dihydroxy-4'-hydroxymethyl-cyclopent-4'-enyl]-8-chloroadenine
(-)-9-[5'-chloro-trans-2',trans-3'-dihydroxy-4'-hydroxymethyl-cyclopent-4'-enyl]-3-deazaadenine
(-)-9-[5'-chloro-trans-2',trans-3'-dihydroxy-4'-hydroxymethyl-cyclopent-4'-enyl]-6-N-methyladenine
(-)-9-[5'-chloro-trans-2',trans-3'-dihydroxy-4'-hydroxymethyl-cyclopent-4'-enyl]-2-chloroadenine
(-)-9-[5'-chloro-cis-2',trans-3'-dihydroxy-cyclopent-4'-enyl]adenine
(-)-9-[5'-chloro-trans-2',trans-3'-dihydroxy-cyclopent-4'-enyl]-8-chloroadenine
(-)-9-[5′-chloro-trans-2′,trans-3′-dihydroxy-cyclopent-4′-enyl]-3-deazaadenine
(-)-9-[5′-chloro-trans-2′,trans-3′-dihydroxy-cyclopent-4′-enyl]-6-N-methyladenine
(-)-9-[5′-chloro-trans-2′,trans-3′-dihydroxy-cyclopent-4′-enyl]-2-chloroadenine.

## EXAMPLE 2

### (-)-9-(5′-FLUORO-TRANS-2′,TRANS-3′-DIHYDROXY-CYCLOPENT-4′-ENYL)ADENINE

Step a: (-)-2,3-(Cyclohexylidenedioxy)-4-cyclopentenone

Prepare the title compound according to the procedure of Borcherding et al., J. Org. Chem. **1987**, 52, 5457.

Step b: (-)-5-Fluoro-2,3-(cyclohexylidenedioxy)-4-cyclopentenone

Prepare the title compound according to a modified procedure of Merritt and Stevens, J. Am. Chem. Soc. **1966**, 88, 1822. To a solution of (-)-2,3-(cyclohexylidenedioxy)-4-cyclopentenone (1.94 g, 10 mmol) in trichlorofluoromethane (20ml) which has been degassed with nitrogen, add 4A molecular sieves and cool to -78°C. Slowly meter-in 5% fluorine in nitrogen through a NaF drying tube until 10 mmol is added. Dilute the reaction mixture with chloroform (20ml) and purify by flash chromatography using a silica gel column and eluting with chloroform. Evaporate the solvent to dryness to yield a solid. Treat the solid with sodium methoxide in methanol [ prepared by reacting sodium metal (400mg, 20 mmol) with methanol (20 ml)] at room temperature for about 16 hours. Neutralize the reaction mixture with dilute aqueous acetic acid and evaporate the solvent to yield an oil. Purify the title compound by flash chromatography using a silica gel column and eluting sequentially with dichloromethane:cyclohexane (1:1) followed by dichloromethane. Evaporate the fractions to dryness to yield a solid.

Step c: (-)-5-Fluoro-2,3-(cyclohexylidenedioxy)-4-cyclopenten-1-ol

Treat the (-)-5-fluoro-2,3-(cyclohexylidenedioxy-4-cyclopentenone (2.23 g, 10.5 mmol) as described for step c in Example 1 to obtain the title compound as a colorless liquid.

Step d: (-)-5-Fluoro-2,3-(cyclohexylidenedioxy)-1-[p-tolylsulfonyl)oxy]cyclopent-4-ene

Treat the (-)-5-fluoro-2,3-(cyclohexylidenedioxy)-4-cyclopenten-1-ol (2.14 g, 10 mmol) as described for step d in Example 1 to obtain the title compound as a white crystalline solid.

Step e: (-)-9-[5′-Fluoro-2′,3′-(cyclohexylidenedioxy) cyclopent-4′-enyl]adenine

Treat the (-)-5-fluoro-2,3-(cyclohexylidenedioxy)-1-[p-tolylsulfonyl)oxy]cyclopent-4-ene (1.50 g, 4.1 mmol) as described for step e in Example 1 to obtain the title compound as a white crystalline solid.

Step f: (-)-9-[5′-Fluoro-trans-2′,trans-3′-dihydroxycyclopent-4′-enyl]adenine

Treat the (-)-9-[5′-fluoro-2′,3′-(cyclohexylidenedioxy) cyclopent-4′-enyl]adenine (331 mg, 1 mmol) as described for step f in Example 1 to obtain the title compound as a white crystalline solid.

In like manner, (-)-9-[5'-fluoro-trans-2',trans-3'-dihydroxy-4'-hydroxymethyl-cyclopent-4'-enyl]adenine can be prepared from (-)-2,3-O-(isopropylidenedioxy)-4-benzyloxymethyl-4-cyclopentenone, which in turn is prepared from ribonolactone as described by Lim and Marques in Tetrahedron Lett. **1983**, 24, 5559. In step f, de-blocking can be accomplished by treatment of the (-)-9-[5'-fluoro-2',3'-O-(isopropylidenedioxy)-4'-benzyloxymethyl-cyclopent-4'-enyl]adenine with $BBr_3$ according to standard procedures.

The following specific compounds can be made by procedures analogous to those described above in Example 2:

(-)-9-[5'-fluoro-trans-2',trans-3'-dihydroxy-4'-(α-hydroxyethyl)-cyclopent-4'-enyl]adenine
(-)-9-[5'-fluoro-cis-2',trans-3'-dihydroxy-4'-hydroxymethyl-cyclopent-4'-enyl]adenine
(-)-9-[5'-fluoro-trans-2',trans-3'-dihydroxy-4'-hydroxymethyl-cyclopent-4'-enyl]-8-chloroadenine
(-)-9-[5'-fluoro-trans-2',trans-3'-dihydroxy-4'-hydroxymethyl-cyclopent-4'-enyl]-3-deazaadenine
(-)-9-[5'-fluoro-trans-2',trans-3'-dihydroxy-4'-hydroxymethyl-cyclopent-4'-enyl]-6-N-methyladenine
(-)-9-[5'-fluoro-trans-2',trans-3'-dihydroxy-4'-hydroxymethyl-cyclopent-4'-enyl]-2-chloroadenine
(-)-9-[5'-fluoro-cis-2',trans-3'-dihydroxy-cyclopent-4'-enyl]adenine
(-)-9-[5′-fluoro-trans-2′,trans-3′-dihydroxy-cyclopent-4′-enyl]-8-chloroadenine
(-)-9-[5′-fluoro-trans-2′,trans-3′-dihydroxy-cyclopent-4′-enyl]-3-deazaadenine
(-)-9-[5′-fluoro-trans-2′,trans-3′-dihydroxy-cyclopent-4′-enyl]-6-N-methyladenine
(-)-9-[5′-fluoro-trans-2′,trans-3′-dihydroxy-cyclopent-4′-enyl]-2-chloroadenine.

In another embodiment, the present invention provides a method of inhibiting AdoMet-dependent transmethylation activity in a patient in need thereof which comprises administration of a compound of the formula (1) in an effective inhibitory amount. The term "effective inhibitory amount" refers to an amount sufficient to inhibit the AdoMet-dependent transmethylation activity after single or multiple dose administration.

As used herein, the term "patient" refers to a warm-blooded animal such as a mammal which is afflicted with a particular disease state. It is understood that dogs, cats, rats, mice, horses, bovine cattle, sheep, and humans are examples of animals within the scope of the meaning of the term.

The compounds of formula (1) are believed to exert their inhibitory effect on AdoMet-dependent transmethylation by inhibition of AdoHcy Hydrolase thereby providing an increase in tissue levels of AdoHcy which in turn provides feedback inhibition of AdoMet-dependent transmethylation. However, it is understood that the present invention is not limited by any particular theory or proposed mechanism to explain its effectiveness in an end-use application.

As is well known and appreciated by those skilled in the art, various disease states, such as certain neoplastic disease states and viral infections, are characterized by excessive Adomet-dependent transmethylation activity. As used herein, the term "excessive" means a level of activity which allows the disease state to progress.

More specifically, the present invention provides a method for the treatment of a patient afflicted with a neoplastic disease state which is characterized by excessive AdoMet dependent transmethylation activity comprising the administration of an effective antineoplastic amount of a compound of formula (1). The term "neoplastic disease state" as used herein refers to an abnormal state or condition characterized by rapidly proliferating cell growth or neoplasm. Neoplastic disease states which are characterized by an excessive AdoMet-dependent transmethylation activity and for which treatment with a compound of formula (1) will be particularly useful include: Leukemias such as, but not limited to, acute lymphoblastic, chronic lymphocytic, acute myeloblastic and chronic myelocytic; Carcinomas, such as, but not limited to, those of the cervix, oesophagus, stomach, small intestines, colon and lungs; Sarcomas, such as, but not limited to, osteoma, osteosarcoma, lipoma, liposarcoma, hemangioma and hemangiosarcoma; Melanomas, including amelanotic and melanotic; and mixed types of neoplasias such as, but not limited to, carcinosarcoma, lymphoid tissue type, follicular reticulum, cell sarcoma and Hodgkins Disease.

An effective antineoplastic amount of a compound of formula (1) refers to an amount which is effective, upon single or multiple dose administration to the patient, in controlling the growth of the neoplasm or in

prolonging the survivability of the patient beyond that expected in the absence of such treatment. As used herein, "controlling the growth" of the neoplasm refers to slowing, interrupting, arresting or stopping its growth and metastases and does not necessarily indicate a total elimination of the neoplasm.

In addition, the present invention provides a method for the treatment of a patient afflicted with a viral infection which is characterized by excessive AdoMet-dependent transmethylation activity comprising the administration of an effective antiviral amount of a compound of formula (1). The term "viral infection" as used herein refers to an abnormal state or condition characterized by viral transformation of cells, viral replication and proliferation. Viral infections which are characterized by an excessive AdoMet dependent transmethylation activity and for which treatment with a compound of formula (1) will be particularly useful include: Retroviruses such as, but not limited to, HTLV-I, HTLV-II, human immunodeficiency viruses, HTLV-III (AIDS virus), and the like; RNA viruses such as, but not limited to, influenza type A, B, and C, mumps, measles, rhinovirus, dengue, rubella, rabies, hepatitis virus A, encephalitis virus, and the like; DNA viruses such as, but not limited to, herpes, vaccinia, pappiloma virus (wart), hepatitis virus B, and the like.

An effective antiviral amount of a compound of formula (1) refers to an amount which is effective in controlling the virus. This viral control refers to slowing, interrupting, arresting or stopping the viral transformation of cells or the replication and proliferation of the virus and does not necessarily indicate a total elimination of the virus.

An effective dose can be readily determined by the attending diagnostician, as one skilled in the art, by the use of conventional techniques and by observing results obtained under analogous circumstances. In determining the effective dose, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances.

Effective antineoplastic and antiviral amounts of a compound of formula (1) are expected to vary from about 0.1 milligram per kilogram of body weight per day (mg/kg/day) to about 100 mg/kg/day. Preferred amounts are expected to vary from about 0.5 to about 10 mg/kg/day.

In an additional embodiment, the present invention relates to a method of treating a patient afflicted with a neoplastic disease state or a viral infection comprising administration of an effective antineoplastic or antiviral amount of a compound of formula (1) wherein Q is $NH_2$ in conjunctive therapy with an effective inhibitory amount of an Adenosine Deaminase (ADA) inhibitor. The term "conjunctive therapy" contemplates coadministration of (1) along with an ADA inhibitor at essentially the same time, or treatment of the patient with an ADA inhibitor prior to or after treatment with a compound of formula (1). An effective inhibitory amount of an ADA inhibitor is an amount effective in significantly inhibiting ADA in the patient.

ADA deaminates compounds of formula (1) wherein Q is $NH_2$ and thereby degrades the active compounds to relatively inactive metabolites. When a compound of formula (1) wherein Q is $NH_2$ and an ADA inhibitor are administered in conjunctive therapy, the dose will be less in amount or frequency of administration than that required when the compound of formula (1) is administered alone.

Various pharmaceutically acceptable non-toxic ADA inhibitors can be used including, but not limited to, deoxycoformycin. An effective inhibitory amount of the ADA inhibitor will vary from about 0.05 mg/kg/day to about 0.5 mg/kg/day and preferably will be from about 0.1 mg/kg/day to about 0.3 mg/kg/day. Deoxycoformycin is the preferred ADA inhibitor for use in conjunctive therapy with compounds of formula (1) wherein Q is $NH_2$.

In effecting treatment of a patient afflicted with a disease state described above, a compound of formula (1) can be administered in any form or mode which makes the compound bioavailable in effective amounts, including oral and parenteral routes. For example, compounds of formula (1) can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular characteristics of the compound selected, the disease state to be treated, the stage of the disease, and other relevant circumstances.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. In addition, compounds of formula (1) wherein Q is $NH_2$ can be administered as above in further combination with an ADA inhibitor. The compounds of the invention, while effective themselves, may be formulated and administered in the form of their pharmaceutically acceptable acid addition salts for purposes of stability, convenience of crystallization, increased

solubility and the like.

In another embodiment, the present invention provides a pharmaceutical composition comprising an effective amount of a compound of formula (1) in admixture or otherwise in association with one or more pharmaceutically acceptable carriers or excipients. In addition, the present invention provides a pharmaceutical composition comprising an effective amount of a compound of formula (1) wherein Q is $NH_2$ and an effective ADA inhibitory amount of an ADA inhibitor in admixture or otherwise in association with one or more pharmaceutically acceptable carriers or excipients. The term "effective amounts" as applied to compounds of formula (1) refers to effective inhibitory, antineoplastic, or antiviral amounts as appropriate.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solutions, suspensions, or the like.

The compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, such as intramuscular, intravenous, and subcutaneous, the compounds of the present invention may be incorporated into a solution or suspension. These preparations should contain at least 0.1% of a compound of the invention, but may be varied to be between 0.1 and about 50% of the weight thereof. The amount of the inventive compound present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Any of the above described pharmaceutical compositions containing compounds of formula (1) wherein Q is $NH_2$ may also contain an effective inhibitory amount of an ADA inhibitor in admixture or otherwise in association with the above described ingredients.

As with any group of structurally related compounds which possess a particular generic utility, certain groups and configurations are preferred for compounds of formula (1) in their end-use application.

With respect to the substituent R, compounds wherein R is H and those wherein R is $CH_2OH$ are generally preferred. With respect to the substituent X, compounds wherein X is chlorine and those wherein X is fluorine are generally preferred.

With respect to the substituents $A_1$ and $A_2$, compounds wherein one of $A_1$ and $A_2$ is hydroxy and the other is hydrogen are generally preferred. Compounds wherein $A_1$ is hydrogen and $A_2$ is hydroxy are

especially preferred.

The following are additional preferred embodiments: compounds of formula (1) wherein $Y_1$ is a CH group, compounds of formula (1) wherein $Y_2$ is nitrogen, compounds of formula (1) wherein $Y_3$ is nitrogen or a CH group, and compounds of formula (1) wherein Z is hydrogen. Finally, with respect to Q, those compounds of formula (1) wherein Q is $NH_2$ or $NHCH_3$ are generally preferred with those wherein Q is $NH_2$ being especially preferred.

The following list identifies compounds of the formula (1) and (1a) which are particularly preferred embodiments of the present invention:

(-)-9-[5'-chloro-trans-2',trans-3'-dihydroxy-cyclopent-4'-enyl]adenine
(-)-9-[5'-fluoro-trans-2',trans-3'-dihydroxy-cyclopent-4'-enyl]adenine
(-)-9-[5'-chloro-trans-2',trans-3'-dihydroxy-4'-hydroxymethyl-cyclopent-4'-enyl]adenine
(-)-9-[5'-fluoro-trans-2',trans-3'-dihydroxy-4'-hydroxymethyl-cyclopent-4'-enyl]adenine
(-)-9-[5'-chloro-trans-2',trans-3'-dihydroxy-cyclopent-4'-enyl]-3-deazaadenine
(-)-9-[5'-fluoro-trans-2',trans-3'-dihydroxy-cyclopent-4'-enyl]-3-deazaadenine
(-)-9-[5'-chloro-trans-2',trans-3'-dihydroxy-4'-hydroxymethyl-cyclopent-4'-enyl]-3-deazaadenine
(-)-9-[5'-fluoro-trans-2',trans-3'-dihydroxy-4'-hydroxymethyl-cyclopent-4'-enyl]-3-deazaadenine.

## Claims

**1.** A neplanocin derivative of the formula

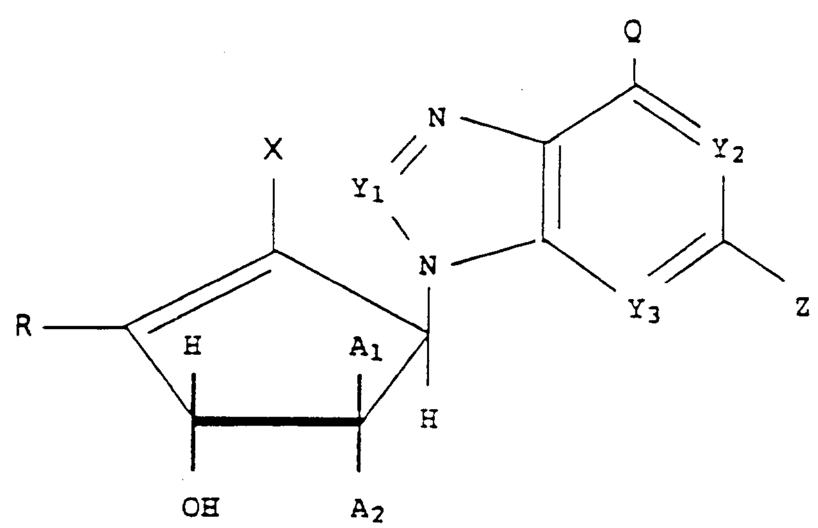

wherein

| | |
|---|---|
| R | is hydrogen or a $C_1$-$C_4$ alkyl optionally substituted with a hydroxy group, |
| X | is halogen, |
| $A_1$ and $A_2$ | are each independently hydrogen, halogen, or hydroxy with the provisos that where $A_1$ is hydroxy, $A_2$ is hydrogen, and that where $A_2$ is hydroxy, $A_1$ is hydrogen, |
| $Y_1$ | is nitrogen, a CH group, a CF group, a CCl group, a CBr group or a $CNH_2$ group, |
| $Y_2$ and $Y_3$ | are each independently nitrogen or a CH group, |
| Q | is $NH_2$, NHOH, $NHCH_3$, or hydrogen, and |
| Z | is hydrogen, halogen, or $NH_2$; |

and pharmaceutically-acceptable salts thereof.

**2.** A compound of Claim 1 wherein $A_2$ is hydroxy.

**3.** A compound of Claim 2 wherein X is fluorine.

**4.** A compound of Claim 2 wherein X is chlorine.

**5.** A compound of Claim 3 or 4 wherein $Y_2$ is nitrogen.

**6.** A compound of Claim 3 or 4 wherein $Y_3$ is nitrogen.

**7.** A compound of Claim 3 or 4 wherein Z is hydrogen.

**8.** A compound of Claim 3 or 4 wherein R is hydrogen.

**9.** A compound of Claim 3 or 4 wherein R is hydroxymethyl.

**10.** The compound of Claim 1 wherein the compound is (-)-9-[5'-chloro-trans-2',trans-3'-dihydroxy-cyclopent-4'-enyl]adenine,
(-)-9-[5'-fluoro-trans-2',trans-3'-dihydroxy-cyclopent-4'-enyl]adenine,
(-)-9-[5'-chloro-trans-2',trans-3'-dihydroxy-4'-hydroxymethyl-cyclopent-4'-enyl]adenine,
(-)-9-[5'-fluoro-trans-2',trans-3'-dihydroxy-4'-hydroxymethyl-cyclopent-4'-enyl]adenine,
(-)-9-[5'-chloro-trans-2',trans-3'-dihydroxy-cyclopent-4'-enyl]-3-deazaadenine,
(-)-9-[5'-fluoro-trans-2',trans-3'-dihydroxy-cyclopent-4'-enyl]-3-deazaadenine,
(-)-9-[5'-chloro-trans-2',trans-3'-dihydroxy-4'-hydroxymethyl-cyclopent-4'-enyl]-3-deazaadenine,
(-)-9-[5'-fluoro-trans-2',trans-3'-dihydroxy-4'-hydroxymethyl-cyclopent-4'-enyl]-3-deazaadenine,
(-)-9-[5'-chloro-trans-2',trans-3'-dihydroxy-4'-($\alpha$-hydroxyethyl)cyclopent-4'-enyl]adenine,
(-)-9-[5'-chloro-cis-2',trans-3'-dihydroxy-4'-hydroxymethyl-cyclopent-4'-enyl]adenine,
(-)-9-[5'-chloro-trans-2',trans-3'-dihydroxy-4'-hydroxymethyl-cyclopent-4'-enyl]-8-chloroadenine,
(-)-9-[5'-chloro-trans-2',trans-3'-dihydroxy-4'-hydroxymethyl-cyclopent-4'-enyl]-6-N-methyladenine,
(-)-9-[5'-chloro-trans-2',trans-3'-dihydroxy-4'-hydroxymethyl-cyclonent-4'-enyl]-2-chloroadenine,
(-)-9-[5'-chloro-cis-2',trans-3'-dihydroxy-cyclopent-4'-enyl]adenine,
(-)-9-[5'-chloro-trans-2',trans-3'-dihydroxy-cyclopent-4'-enyl]-8-chloroadenine,
(-)-9-[5'-chloro-trans-2',trans-3'-dihydroxy-cyclopent-4'-enyl]-6-N-methyladenine, or
(-)-9-[5'-chloro-trans-2',trans-3'-dihydroxy-cyclopent-4'-enyl]-2-chloroadenine.

**11.** Use of a compound of any of Claims 1 to 10 for the preparation of a pharmaceutical composition for inhibiting AdoMet-dependent transmethylation activity in a patient in need thereof.

**12.** Use of a compound of any of Claims 1 to 10 for the preparation of a pharmaceutical composition for the treatment of a patient afflicted with a neoplastic disease state.

**13.** Use of a compound of any of Claims 1 to 10 for the preparation of a pharmaceutical composition for the treatment of a patient afflicted with a viral infection.

**14.** Use of a compound of any of Claims 1 to 10 for the preparation of a pharmaceutical composition for controlling the growth of mammalian cells characterized by the presence of a transmethylation dependent disease state.

**15.** Use according to any of claims 12, 13 or 14 wherein the neplanocin derivative of any of Claims 1 to 10 is used together with an effective inhibitory amount of an ADA inhibitor while the two effective compounds are either to be coadministered or separate sequential administration is contemplated.

**16.** A pharmaceutical composition for inhibiting AdoMet-dependent transmethylation activity in a patient in need thereof comprising an effective inhibitory amount of a compound of any of Claims 1 to 10.

**17.** A pharmaceutical composition for treating a patient afflicted with a neoplastic disease state comprising an effective antineoplastic amount of a compound of any of Claims 1 to 10.

**18.** A pharmaceutical composition for treating a patient afflicted with a viral infection comprising an effective antiviral amount of a compound of any of Claims 1 to 10.

**19.** A pharmaceutical composition for controlling the growth of mammalian cells characterized by the presence of a transmethylation dependent disease state comprising an effective inhibitory amount of a compound of any of claims 1 to 10.

**20.** A pharmaceutical composition for any of the uses according to Claims 17, 18 or 19, characterized by the additional presence of an effective inhibitory amount of an ADA inhibitor.

**21.** A pharmaceutical composition comprising a compound as defined in any of Claims 1 to 10.

**22.** A method for the preparation of a pharmaceutical composition as defined in any of Claims 16 to 20 comprising combining a pharmaceutically acceptable carrier with a compound as defined in any of Claims 1 to 10.

**23.** A method for the preparation of a compound as defined in any of Claims 1 to 10 which comprises treating with an acid a compound of the formula

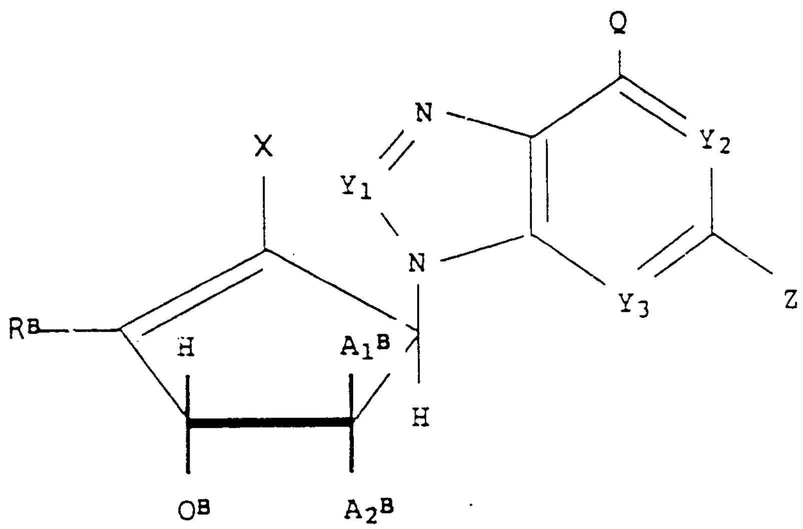

wherein X, $Y_1$, $Y_2$, $Y_3$, Z and Q are as defined,
$O^B$ represents a protected hydroxy group,
$A_1{}^B$ and $A_2{}^B$ represent protected hydroxy groups when $A_1$ or $A_2$ are hydroxy and represent $A_1$ or $A_2$ when $A_1$ or $A_2$ are other than hydroxy, and
$R^B$ represents a $C_1$-$C_4$ alkyl bearing a protected hydroxy group when R is a $C_1$-$C_4$ alkyl bearing a hydroxy group and represents R when R is other than a $C_1$-$C_4$ alkyl bearing a hydroxy group,
and optionally converting the compound with a pharmaceutically acceptable acid to the acid addition salt.

## Patentansprüche

1. Neplanocinderivat der Formel:

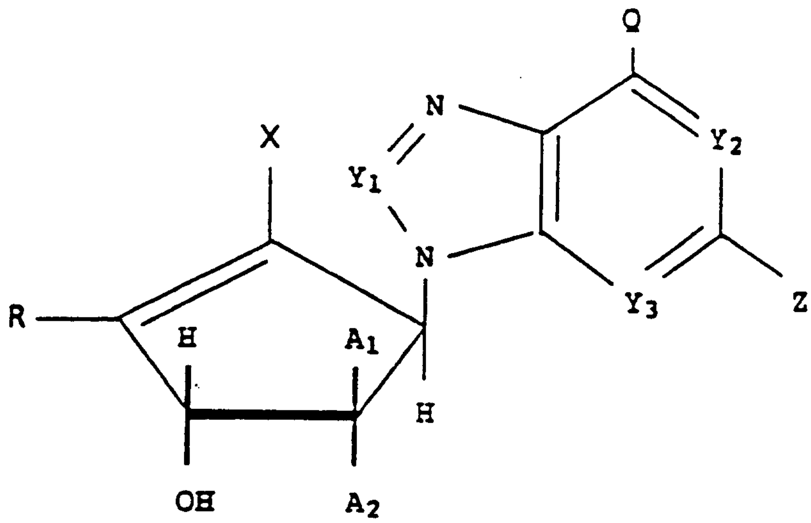

in der

| | |
|---|---|
| R | ein Wasserstoffatom oder einen $C_1$-$C_4$-Alkylrest bedeutet, der gegebenenfalls mit einer Hydroxygruppe substituiert ist, |
| X | ein Halogenatom darstellt, |
| $A_1$ und $A_2$ | jeweils unabhängig voneinander ein Wasserstoff- oder Halogenatom oder eine Hydroxygruppe bedeuten, mit den Maßgaben, daS $A_2$ ein Wasserstoffatom darstellt, wenn $A_1$ eine Hydroxygruppe bedeutet, und daß $A_1$ ein Wasserstoffatom darstellt, wenn $A_2$ eine Hydroxygruppe bedeutet, |
| $Y_1$ | ein Stickstoffatom, eine CH-Gruppe, eine CF-Gruppe, eine CCl-Gruppe, eine CBr-Gruppe oder eine $CNH_2$-Gruppe darstellt, |
| $Y_2$ und $Y_3$ | jeweils unabhängig ein Stickstoffatom oder eine CH-Gruppe bedeuten, |
| Q | eine $NH_2$-, NHOH- oder $NHCH_3$-Gruppe oder ein Wasserstoffatom darstellt und |
| Z | ein Wasserstoff- oder Halogenatom oder eine $NH_2$-Gruppe bedeutet, |

und pharmazeutisch verträgliche Salze davon.

2. Verbindung nach Anspruch 1, wobei $A_2$ eine Hydroxygruppe darstellt.

3. Verbindung nach Anspruch 2, wobei X ein Fluoratom bedeutet.

4. Verbindung nach Anspruch 2, wobei X ein Chloratom darstellt.

5. Verbindung nach Anspruch 3 oder 4, wobei $Y_2$ ein Stickstoffatom bedeutet.

6. Verbindung nach Anspruch 3 oder 4, wobei $Y_3$ ein Stickstoffatom darstellt.

7. Verbindung nach Anspruch 3 oder 4, wobei Z ein Wasserstoffatom bedeutet.

8. Verbindung nach Anspruch 3 oder 4, wobei R ein Wasserstoffatom darstellt.

9. Verbindung nach Anspruch 3 oder 4, wobei R eine Hydroxymethylgruppe bedeutet.

10. Verbindung nach Anspruch 1, wobei die Verbindung
(-)-9-[5'-Chlor-trans-2',trans-3'-dihydroxycyclopent-4'-enyl]adenin,
(-)-9-[5'-Fluor-trans-2',trans-3'-dihydroxycyclopent-4'-enyl]adenin,
(-)-9-[5'-Chlor-trans-2',trans-3'-dihydroxy-4'-hydroxymethylcyclopent-4'-enyl]adenin,

(-)-9-[5'-Fluor-trans-2',trans-3'-dihydroxy-4'-hydroxymethylcyclopent-4'-enyl]adenin,
(-)-9-[5'-Chlor-trans-2',trans-3'-dihydroxycyclopent-4'-enyl]-3-deazaadenin,
(-)-9-[5'-Fluor-trans-2',trans-3'-dihydroxycyclopent-4'-enyl]-3-deazaadenin,
(-)-9-[5'-Chlor-trans-2',trans-3'-dihydroxy-4'-hydroxymethylcyclopent-4'-enyl]-3-deazaadenin,
(-)-9-[5'-Fluor-trans-2',trans-3'-dihydroxy-4'-hydroxymethylcyclopent-4'-enyl]-3-deazaadenin,
(-)-9-[5'-Chlor-trans-2',trans-3'-dihydroxy-4'-(α-hydroxyethyl)cyclopent-4'-enyl]adenin,
(-)-9-[5'-Chlor-cis-2',trans-3'-dihydroxy-4'-hydroxymethylcyclopent-4'-enyl]adenin,
(-)-9-[5'-Chlor-trans-2',trans-3'-dihydroxy-4'-hydroxymethylcyclopent-4'-enyl]-8-chloradenin,
(-)-9-[5'-Chlor-trans-2',trans-3'-dihydroxy-4'-hydroxymethylcyclopent-4'-enyl]-6-N-methyladenin,
(-)-9-[5'-Chlor-trans-2',trans-3'-dihydroxy-4'-hydroxymethylcyclopent-4'-enyl]-2-chloradenin,
(-)-9-[5'-Chlor-cis-2',trans-3'-dihydroxycyclopent-4'-enyl]adenin,
(-)-9-[5'-Chlor-trans-2',trans-3'-dihydroxycyclopent-4'-enyl]-8-chloradenin,
(-)-9-[5'-Chlor-trans-2',trans-3'-dihydroxycyclopent-4'-enyl]-6-N-methyladenin, oder
(-)-9-[5'-Chlor-trans-2',trans-3'-dihydroxycyclopent-4'-enyl]-2-chloradenin ist.

**11.** Verwendung einer Verbindung nach einem der Ansprüche 1 bis 10 zur Herstellung eines Arzneimittels zur Hemmung der AdoMet-abhängigen Transmethylierungswirksamkeit bei einem Patienten, der dessen bedarf.

**12.** Verwendung einer Verbindung nach einem der Ansprüche 1 bis 10 zur Herstellung eines Arzneimittels zur Behandlung eines Patienten, der von einem neoplastischen Erkrankungszustand befallen ist.

**13.** Verwendung einer Verbindung nach einem der Ansprüche 1 bis 10 zur Herstellung eines Arzneimittels zur Behandlung eines Patienten, der von einer Virusinfektion befallen ist.

**14.** Verwendung einer Verbindung nach einem der Ansprüche 1 bis 10 zur Herstellung eines Arzneimittels zur Bekämpfung des Wachstums von Säugerzellen, die durch die Anwesenheit eines transmethylierungsabhängigen Erkrankungszustands gekennzeichnet sind.

**15.** Verwendung nach einem der Ansprüche 12, 13 oder 14, wobei das Neplanocinderivat nach einem der Ansprüche 1 bis 10 zusammen mit einer wirksamen hemmenden Menge eines ADA-Hemmstoffs verwendet wird, während die zwei wirksamen Verbindungen entweder zusammen zu verabreichen sind oder eine getrennte aufeinanderfolgende Verabreichung erwogen wird.

**16.** Arzneimittel zur Hemmung der AdoMet-abhängigen Transmethylierungswirksamkeit bei einem Patienten, der dessen bedarf, das eine wirksame hemmende Menge einer Verbindung nach einem der Ansprüche 1 bis 10 umfaßt.

**17.** Arzneimittel zur Behandlung eines Patienten, der von einem neoplastischen Erkrankungszustand befallen ist, das eine wirksame antineoplastische Menge einer Verbindung nach einem der Ansprüche 1 bis 10 umfaßt.

**18.** Arzneimittel zur Behandlung eines Patienten, der von einer Virusinfektion befallen ist, das eine wirksame antivirale Menge einer Verbindung nach einem der Ansprüche 1 bis 10 umfaßt.

**19.** Arzneimittel zur Bekämpfung des Wachstums von Säugerzellen, die durch die Anwesenheit eines transmethylierungsabhängigen Erkrankungszustands gekennzeichnet sind, das eine wirksame hemmende Menge einer Verbindung nach einem der Ansprüche 1 bis 10 umfaßt.

**20.** Arzneimittel für eine der Verwendungen nach den Ansprüchen 17, 18 oder 19, das durch die zusätzliche Anwesenheit einer wirksamen hemmenden Menge eines ADA-Hemmstoffs gekennzeichnet ist.

**21.** Arzneimittel, das eine Verbindung nach einem der Ansprüche 1 - 10 umfaßt.

**22.** Verfahren zur Herstellung eines Arzneimittels nach einem der Ansprüche 16 - 20, das das Kombinieren eines pharmazeutisch verträglichen Trägers mit einer Verbindung nach einem der Ansprüche 1 - 10 umfaßt.

**23.** Verfahren zur Herstellung einer Verbindung nach einem der Ansprüche 1 - 10, das die Behandlung einer Verbindung der Formel

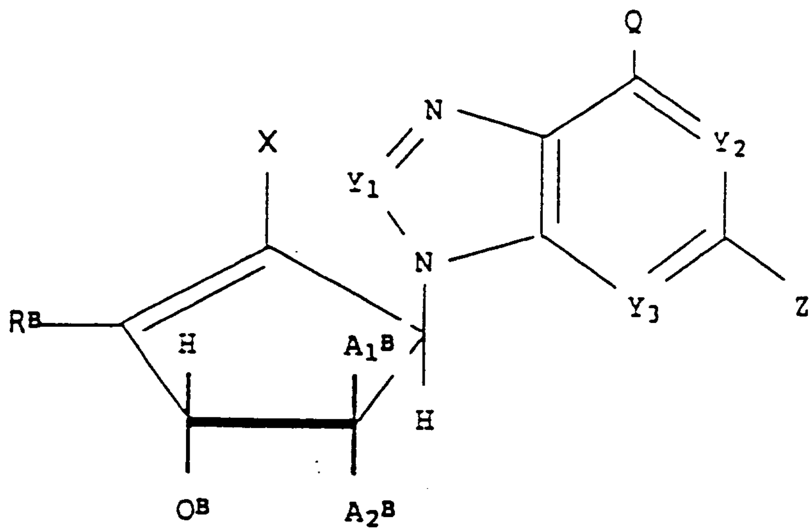

in der

X, $Y_1$, $Y_2$, $Y_3$, Z und Q wie definiert sind,

$O^B$ eine geschützte Hydroxygruppe darstellt,

$A_1{}^B$ und $A_2{}^B$ geschützte Hydroxygruppen darstellen, wenn $A_1$ oder $A_2$ Hydroxygruppen bedeuten, und $A_1$ oder $A_2$ darstellen, wenn $A_1$ oder $A_2$ etwas anderes als Hydroxygruppen bedeuten, und

$R^B$ einen $C_1$-$C_4$-Alkylrest darstellt, der eine geschützte Hydroxygruppe trägt, wenn R einen $C_1$-$C_4$-Alkylrest bedeutet, der eine Hydroxygruppe trägt, und R darstellt, wenn R etwas anderes als ein $C_1$-$C_4$-Alkylrest ist, der eine Hydroxygruppe trägt,

mit einer Säure und gegebenenfalls die Umwandlung der Verbindung mit einer pharmazeutisch verträglichen Säure zum Säureadditionssalz umfaßt.

**Revendications**

**1.** Dérivé de la néplanocine de formule

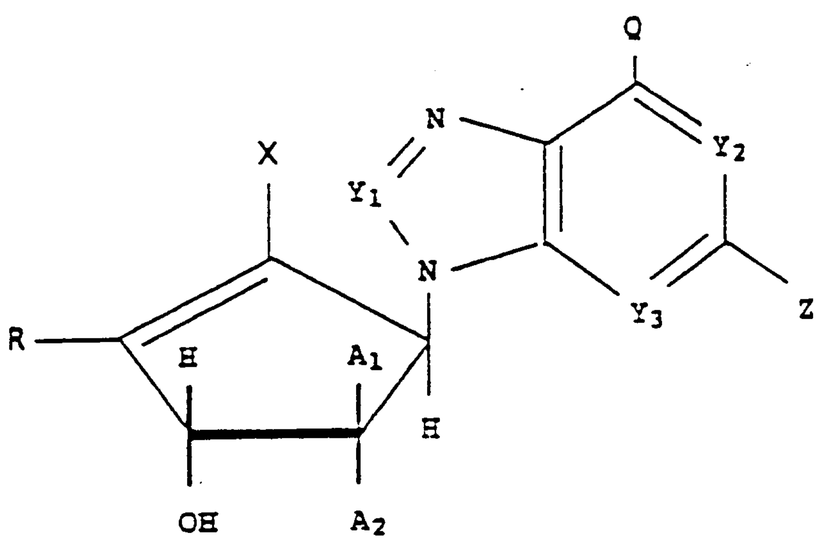

dans laquelle

R est l'hydrogène ou un alkyle en $C_1$-$C_4$ éventuellement substitué avec un groupe hydroxyle,

X est un halogène,
$A_1$ et $A_2$ sont chacun indépendamment l'hydrogène, un halogène ou un hydroxyle à condition que lorsque $A_1$ est hydroxyle, $A_2$ soit l'hydrogène et que lorsque $A_2$ est hydroxyle, $A_1$ soit l'hydrogène,
$Y_1$ est l'azote, un groupe CH, un groupe CF, un groupe CCl, un groupe CBr ou un groupe $CNH_2$,
$Y_2$ et $Y_3$ sont chacun indépendamment l'azote ou un groupe CH,
Q est $NH_2$, NHOH, $NHCH_3$ ou l'hydrogène, et
Z est l'hydrogène, un halogène, ou $NH_2$ ;
et ses sels pharmaceutiquement acceptables.

**2.** Composé selon la revendication 1, dans lequel $A_2$ est hydroxyle.

**3.** Composé selon la revendication 2, dans lequel X est le fluor.

**4.** Composé selon la revendication 2, dans lequel X est le chlore.

**5.** Composé selon la revendication 3 ou 4, dans lequel $Y_2$ est l'azote.

**6.** Composé selon la revendication 3 ou 4, dans lequel $Y_3$ est l'azote.

**7.** Composé selon la revendication 3 ou 4, dans lequel Z est l'hydrogène.

**8.** Composé selon la revendication 3 ou 4, dans lequel R est l'hydrogène.

**9.** Composé selon la revendication 3 ou 4, dans lequel R est hydroxyméthyle.

**10.** Composé selon la revendication 1, dans lequel le composé est
la (-)-9-[5'-chloro-trans-2',trans-3'-dihydroxycyclopent-4'-ényl]adénine,
la (-)-9-[5'-fluoro-trans-2',trans-3'-dihydroxycyclopent-4'-ényl]adénine,
la (-)-9-[5'-chloro-trans-2',trans-3'-dihydroxy-4'-hydroxyméthylcyclopent-4'-ényl]adénine,
la (-)-9-[5'-fluoro-trans-2',trans-3'-dihydroxy-4'-hydroxyméthylcyclopent-4'-ényl]adénine,
la (-)-9-[5'-chloro-trans-2',trans-3'-dihydroxycyclopent-4'-ényl]-3-déazaadénine,
la (-)-9-[5'-fluoro-trans-2',trans-3'-dihydroxycyclopent-4'-ényl]-3-déazaadénine,
la (-)-9-[5'-chloro-trans-2',trans-3'-dihydroxy-4'-hydroxyméthylcyclopent-4'-ényl]-3-déazaadénine,
la (-)-9-[5'-fluoro-trans-2',trans-3'-dihydroxy-4'-hydroxyméthylcyclopent-4'-ényl]-3-déazaadénine,
la (-)-9-[5'-chloro-trans-2',trans-3'-dihydroxy-4'-(α-hydroxyéthylcyclopent-4'-ényl]adénine,
la (-)-9-[5'-chloro-cis-2',trans-3'-dihydroxy-4'-hydroxyméthylcyclopent-4'-ényl]adénine,
la (-)-9-[5'-chloro-trans-2',trans-3'-dihydroxy-4'-hydroxyméthylcyclopent-4'-ényl]-8-chloroadénine,
la (-)-9-[5'-chloro-trans-2',trans-3'-dihydroxy-4'-hydroxyméthylcyclopent-4'-ényl]-6-N-méthyladénine,
la (-)-9-[5'-chloro-trans-2',trans-3'-dihydroxy-4'-hydroxyméthylcyclopent-4'-ényl]-2-chloroadénine,
la (-)-9-[5'-chloro-cis-2',trans-3'-dihydroxycyclopent-4'-ényl]adénine,
la (-)-9-[5'-chloro-trans-2',trans-3'-dihydroxycyclopent-4'-ényl]-8-chloroadénine,
la (-)-9-[5'-chloro-trans-2',trans-3'-dihydroxycyclopent-4'-ényl]-6-N-méthyladénine ou
la (-)-9-[5'-chloro-trans-2',trans-3'-dihydroxycyclopent-4'-ényl]-2-chloroadénine.

**11.** Utilisation d'un composé selon l'une quelconque des revendications 1 à 10 pour la préparation d'une composition pharmaceutique pour inhiber l'activité de transméthylation dépendante de l'AdoMet chez un patient qui en a besoin.

**12.** Utilisation d'un composé selon l'une quelconque des revendications 1 à 10 pour la préparation d'une composition pharmaceutique pour le traitement d'un patient souffrant d'un état morbide néoplasique.

**13.** Utilisation d'un composé selon l'une quelconque des revendications 1 à 10 pour la préparation d'une composition pharmaceutique pour le traitement d'un patient souffrant d'une infection virale.

**14.** Utilisation d'un composé selon l'une quelconque des revendications 1 à 10 pour la préparation d'une composition pharmaceutique pour contrôler la croissance de cellules de mammifère caractérisées par la présence d'un état morbide dépendant de la transméthylation.

**15.** Utilisation selon l'une quelconque des revendications 12, 13 ou 14 dans laquelle le dérivé de la néplanocine selon l'une quelconque des revendications 1 à 10 est utilisé en même temps qu'une quantité inhibitrice efficace d'un inhibiteur de l'ADA tandis que les deux composés efficaces sont destinés à être co-administrés ou qu'une administration successive séparée est envisagée.

**16.** Composition pharmaceutique pour inhiber l'activité de transméthylation dépendante de l'AdoMet chez un patient qui en a besoin, comprenant une quantité inhibitrice efficace d'un composé selon l'une quelconque des revendications 1 à 10.

**17.** Composition pharmaceutique pour traiter un patient souffrant d'un état morbide néoplasique, comprenant une quantité antinéoplasique efficace d'un composé selon l'une quelconque des revendications 1 à 10.

**18.** Composition pharmaceutique pour traiter un patient souffrant d'une infection virale, comprenant une quantité antivirale efficace d'un composé selon l'une quelconque des revendications 1 à 10.

**19.** Composition pharmaceutique pour contrôler la croissance de cellules de mammifère caractérisées par la présence d'un état morbide dépendant de la transméthylation, comprenant une quantité inhibitrice efficace d'un composé selon l'une quelconque des revendications 1 à 10.

**20.** Composition pharmaceutique pour l'une quelconque des utilisations selon les revendications 17, 18 ou 19, caractérisée par la présence supplémentaire d'une quantité inhibitrice efficace d'un inhibiteur de l'ADA.

**21.** Composition pharmaceutique comprenant un composé tel que défini dans l'une quelconque des revendications 1 à 10.

**22.** Procédé de préparation d'une composition pharmaceutique selon l'une quelconque des revendications 16 à 20, comprenant la combinaison d'un véhicule pharmaceutiquement acceptable avec un composé selon l'une quelconque des revendications 1 à 10.

**23.** Procédé de préparation d'un composé selon l'une quelconque des revendications 1 à 10, qui comprend le traitement avec un acide d'un composé de formule

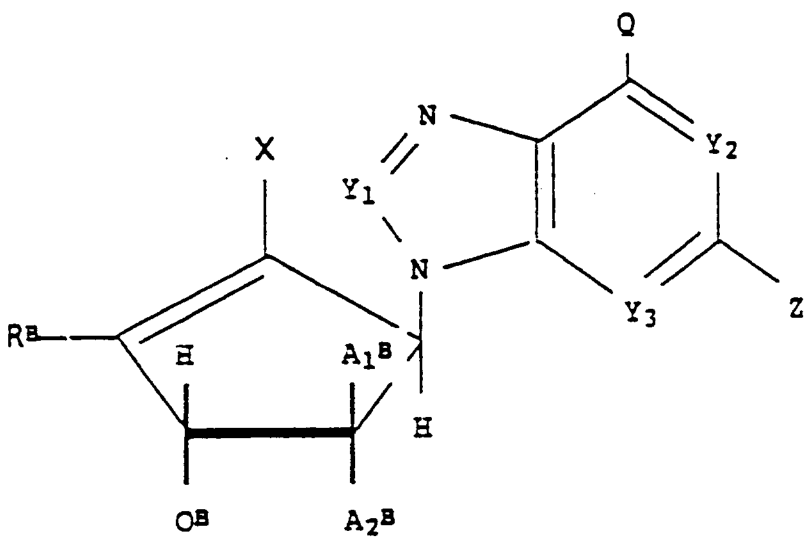

dans laquelle X, $Y_1$, $Y_2$, $Y_3$, Z et Q sont tels que définis,
$O^B$ représente un groupe hydroxyle protégé,
$A_1B$ et $A_2B$ représentent des groupes hydroxyles protégés lorsque $A_1$ ou $A_2$ est un hydroxyle et représentent $A_1$ ou $A_2$ lorsque $A_1$ ou $A_2$ ne sont pas des hydroxyles, et
$R^B$ représente un alkyle en $C_1$-$C_4$ portant un groupe hydroxyle protégé lorsque R est un alkyle en $C_1$-

$C_4$ portant un groupe hydroxyle et représente R lorsque R n'est pas un alkyle en $C_1$-$C_4$ portant un groupe hydroxyle,
et éventuellement la conversion du composé avec un acide pharmaceutiquement acceptable en le sel d'addition d'acide.